# EUROPEAN PATENT SPECIFICATION

(11) **EP 2 817 010 B1**
(45) Date of publication and mention of the grant of the patent: **08.08.2018**
(21) Application number: 13751898.1
(22) Date of filing: 19.02.2013
(51) Int. Cl.: A61K 31/4192, A61K 31/495, A61P 35/00

(54) **COMPOSITIONS FOR ENHANCING SENSITIVITY OF CYTOTOXIC DRUGS WITH TIMELY COMBINATORIAL THERAPY WITH CARBOXYAMIDOTRIAZOLE OROTATE**
ZUSAMMENSETZUNGEN ZUR VERBESSERUNG DER EMPFINDLICHKEIT VON ZYTOTOXISCHEN MEDIKAMENTEN MIT TEMPORÄRER KOMBINATIONSTHERAPIE MIT CARBOXYAMIDOTRIAZOLOROTAT
COMPOSITIONS D'AMÉLIORATION DE LA SENSIBILITÉ DE MÉDICAMENTS CYTOTOXIQUES AVEC UNE THÉRAPIE COMBINATOIRE OPPORTUNE AVEC DE L'OROTATE DE CARBOXYAMIDOTRIAZOLE

(30) Priority: 21.02.2012 US 201213385449
(43) Date of publication of application: 31.12.2014
(73) Proprietor: Tactical Therapeutics, Inc., New York, NY 10005 (US)
(72) Inventor: KARMALI, Rashida, A., New York, NY 10005 (US)
(74) Representative: Bates, Philip Ian
(86) International application number: PCT/US2013/000039
(87) International publication number: WO 2013/126146

(56) References cited:
- US-A1- 2008 255 035
- US-A1- 2009 291 088
- Anonymous: "NCT01107522 on 2011_10_17", ClinicalTrials.gov Archive, 17 October 2011 (2011-10-17), pages 1-5, XP055195789, Retrieved from the Internet: URL:https://clinicaltrials.gov/archive/NCT 01107522/2011_10_17 [retrieved on 2015-06-15]
- Rashida A Karmali ET AL: "Combinatorial treatment with carboxyamidotriazole- orotate and temozolomide in sc-implanted human LOX IMVI melanoma xenografts", Journal of Solid Tumors, 1 October 2012 (2012-10-01), pages 1925-4067, XP055195573, DOI: 10.5430/jst.v2n5px Retrieved from the Internet: URL:http://www.tacticaltherapeutics.com/wp -content/uploads/2014/06/Journal-of-Solid- Tumors.pdf [retrieved on 2015-06-12]
- R.A. KARMALI, Y. MAXUITENKO, G. GORMAN: "Combinatorial treatment with CTO and temozolomide in sc-implanted human LOX IMVI melanoma", J. CLIN. ONCOLOGY 2012 ASCO ANNUAL MEETING ABSTRACTS, vol. 30, no. 15Suppl, E19006, 20 May 2012 (2012-05-20), XP008176686,
- Y. MAXUITENKO, G. GORMAN, R. KARMALI: "Combinatorial treatment using CTO with paclitxael or paclitaxel orotate in sc-implanted human OVCAR-5 ovarian tumor model.", J. CLIN. ONCOL., vol. 30, no. 15suppl, E15515, 20 May 2012 (2012-05-20), XP008176685,
- CHIARA CORRADO ET AL: "Carboxyamidotriazole-Orotate Inhibits the Growth of Imatinib-Resistant Chronic Myeloid Leukaemia Cells and Modulates Exosomes-Stimulated Angiogenesis", PLOS ONE, vol. 7, no. 8, 3 August 2012 (2012-08-03), page e42310, XP055195851, US ISSN: 1932-6203, DOI: 10.1371/journal.pone.0042310
- KARMALI ET AL.: 'Carboxyamidotriazole Orotate and Cytotoxic Chemotherapy have a Synergistic Effect on Tumor Inhibition in Glioblastoma and Colon Xenograft Mouse Models.' CANCER THERAPY vol. 8, 2011, pages 71 - 79, XP055160506
- GERBER ET AL.: 'Pharmacology and pharmacodynamics of bevacizumab as monotherapy or in combination with cytotoxic therapy in preclinical studies.' CANCER RESEARCH vol. 65, no. 3, 2005, pages 671 - 680, XP002481174
- ALESSANDRO ET AL.: 'Effects of carboxyamidotriazole on in vitro models of imatinib-resistant chronic myeloid leukemia.' JOURNAL OF CELLULAR PHYSIOLOGY vol. 215, no. 1, 2008, pages 111 - 121, XP055160509
- PRASSL ET AL.: 'Striking response with bevacizumab and chemotherapy in a woman with heavily pretreated breast cancer: a case presentation' MEMO-MAGAZINE OF EUROPEAN MEDICAL ONCOLOGY vol. 1, no. 3, 2008, pages 149 - 151, XP055160510
- MATTHEW H. TAYLOR ET AL: "Effect of Carboxyamidotriazole Orotate, a Modulator of Calcium-Dependent Signaling Pathways, on Advanced Solid Tumors", JOURNAL OF CANCER THERAPY, vol. 06, no. 04, 1 January 2015 (2015-01-01), pages 322-333, XP055260414, ISSN: 2151-1934, DOI: 10.4236/jct.2015.64035
- K. VERA: "Dose-dense regimen of temozolomide given every other week in patients with primary central nervous system tumors", ANNALS OF ONCOLOGY., vol. 15, no. 1, 1 January 2004 (2004-01-01), pages 161-171, XP055260456, NL ISSN: 0923-7534, DOI: 10.1093/annonc/mdh003

## Description

### 1. FIELD OF INVENTION

This invention is related to enhancing the sensitivity of cytotoxic chemotherapeutic and biologic drugs (collectively referred to as "cytotoxic" drugs) in malignant tumors by targeting the initial mechanisms of the cytotoxic drugs in the tumor microenvironment and/or the subsequent development of drug resistance, using combinatorial therapy with carboxyamidotriazole orotate (CTO). Such carefully designed combinatorial therapy comprising titrated amounts of the cytotoxic drugs and CTO achieves maximum efficacy without resorting to dose dense strategies which are associated with serious toxicity. Such a combinatorial therapeutic system will improve the quality of life for patients.

### 2. BACKGROUND

Cytotoxic drugs are now used at some time during the course of the treatment of most cancer patients. Cytotoxic drugs can cure some primary and metastatic cancers and be effective in decreasing tumor volume, treating symptoms and even prolonging life in many types of cancers. The starting dose of the cytotoxic drug in cancer patients is based on preclinical evaluation of the therapeutic dose in different tumor cell lines in vitro and in animal tumor modes in vivo as required by the Guidelines from the Food & Drug Administration for Nonclinical Evaluation for Anticancer Pharmaceuticals (ICH S9). In experimental xenograft tumor models the dose-response curve is usually steep in the linear phase, and a reduction in dose when the tumor is in the linear phase of the dose-response curve, almost always results in a loss in the capacity to cure the tumor effectively before a reduction in the antitumor activity is observed. Although xenograft models may not represent the ideal model for human malignancies, the general principles have been applied to the clinical setting. Such empirical modifications in dose represent a major reason for treatment failure in patients with drug-sensitive tumors who are receiving chemotherapy in either the adjuvant or advanced disease setting.

Another reason for treatment failure in patients with drug-sensitive tumors who are receiving a cytotoxic drug is the effect of the cytotoxic drug on the microenvironment of the tumor since the chemotherapy may cause mechanisms that can interfere with its antitumor activity on the tumor itself.

Since the development of chemotherapy in the 1950s and 1960s, which resulted in curative therapeutic strategies for patients with several types of solid tumors and hematologic malignancies, the understanding of genetic changes that can result in drug resistance has provided innovative therapeutic strategies; but unfortunately, these studies have not heretofore, considered the early effect of the chemotherapy on the microenvironment of the tumor in the determination of the sensitivity and effective doses required to achieve treatment success. Carboxyamidotriazole orotate (CTO) is an orotate salt of carboxyamidotriazole (CAI). CAI is an inhibitor of receptor-operated calcium channel-mediated calcium influx, and is shown to have anti-proliferative and anti-invasive functions in several human cancer cell lines, including human glioblastoma cells (Ge et al, 2000). By interrupting calcium mobilization as a second messenger, CAI can inhibit calcium-sensitive signal transduction pathways, including the release of arachidonic acid and its metabolites; nitric oxide release; the generation of inositol phosphates; and tyrosine phosphorylation (Ge et al, 2000; Kohn et al, 1992). CAI inhibits phosphorylation of cellular proteins STATS and CrkL, and induces apoptosis in imatinib mesylate-resistant chronic myeloid leukemia cells by down-regulating bcr-abl (Alessandro et al, 2008). CTO targets the tumors as well as the microenvironment of the tumor and mechanisms that may induce drug resistance or interfere with the antitumor activity.

Currently, the established dosing regimens for cytotoxic drugs have not factored in this interference in sensitivity of the cytotoxic drugs, hence leading to increase in the dose used. Dose-dense strategies are used to achieve tumor shrinkage but these cause severe toxicities in cancer patients and adversely affect their quality of life. There is a critical need to prevent or reduce this interference in sensitivity instead of using dose dense regimens. Chemotherapy with cytotoxic drugs is presently used in four main clinical settings: 1) primary induction treatment for advanced disease or for cancers for which there are no other effective treatment approaches, 2) neoadjuvant treatment for patients who present with localized disease for whom local forms of therapy such as surgery and/or radiation are inadequate by themselves, 3) adjuvant treatment to local methods of treatment, including surgery and/or radiation therapy, and 4) direct instillation into sanctuary sites or by site-directed perfusion of specific regions of the body directly affected by the cancer. Physicians Cancer Chemotherapy Drug Manual, ed, E.Chu, V.T. DeVita, Jr, 2010. Drug resistance may also be caused by malignant cells becoming resistant to the drug and a number of cellular mechanisms are probably involved in altering metabolism of the drug, permeability of the cells to the drug or accelerated elimination of the drug, altered specificity of the inhibited enzyme, or amplification of certain genes involved in resistance to chemotherapy or biologic therapy. This is observed after multiple exposures to the drug as described below.

For example, amplification of the gene encoding dihydrofolate reductase is related to resistance to methotrexate, while amplification of the gene encoding thymidylate synthase is related to resistance to treatment with 5-fluoropyridines.

The therapeutic benefit of temozolomide depends on its ability to alkylate (for example methylate) DNA, which most often occurs at the N-7 or O-6 positions of guanine residues. This methylation damages the DNA and triggers the death of tumor cells. However, some tumor cells are able to repair this type of DNA damage, and therefore diminish the therapeutic efficacy of temozolomide by expressing O-6-methyguanine-DNA methyltransferase (MSMT) or O-6-alkyguanine -DNA alkyltransferase (AGT or AGAT). In some tumors epigenetic silencing of the MGMT/AGT gene prevents the synthesis of this enzyme, and as a consequence such tumors are sensitive to killing by temozolomide. Conversely, the presence of MGMT protein in brain tumors predicts poor response to temozolomide and these patients receive little benefit. But resistance to temozolomide is related to other factors as well. In GBM patients the tumor responds to temozolomide at first but later, even with increased doses, the tumor becomes refractory after a few courses. This suggests that other interfering factors may be reducing the sensitivity of temozolomide to cancer cells.

There is some evidence that changes in the tumor microenvironment induced by doxorubicin may impede its delivery to the tumor target and therefore a combinatorial regimen of doxorubicin and CTO may provide a solution to maintain doxorubicin's sensitivity. Karmali et al. disclose in Cancer Therapy, 8, 2011, 71-79 a combination of the VEGF inhibitor CTO and temozolomide with synergistic activity in a glioblastoma mouse model. In some cases resistance to a drug may be linked to increased production of molecules (e.g., cytokines, calcium channel signaling, molecular signaling) in the tumor microenvironment that interfere with the sensitivity and efficacy of the cytotoxic drugs. Therefore, even the most rationally conceived drug molecule may fail because of mutational changes downstream from its intended target or metabolic features of tumors that never allow the drug to reach its target or that trigger feedback mechanism against the drug molecule.

Therefore a rational approach to cancer drug therapy and development is needed that relies on the empirical evidence of tumor shrinkage with cytotoxic drugs, understanding mechanisms of action of the chemotherapy in the tumor microenvironment in real time, defining new lead structures directed to biochemical and molecular targets and causing the cytotoxic drugs to perform optimally.

### 3. SUMMARY OF THE INVENTION

Hereby provided according to the invention is a composition comprising temozolomide for use in the treatment of melanoma wherein the treatment comprises administration orally of carboxyamidotriazole orotate and wherein the temozolomide is administered orally at a dose of 60 mg/kg/Q4Dx3, and wherein the carboxyamidotriazole orotate is administered at a dose of 342 mg/kg/day. Subject-matter which is not encompassed by the scope of the claims does not form part of the present claimed invention.

### 4. BRIEF DESCRIPTION OF FIGURES

Fig. 1 illustrates the response of SC Implanted LOX IMVI Human Melanoma to Treatment with different doses of CTO.
Fig. 2 illustrates the response of SC Implanted LOX IMVI Human Melanoma to Treatment with different doses of temozolomide
Fig. 3 illustrates the response of SC Implanted LOX IMVI Human Melanoma to Treatment with different doses of CTO with the High Dose of Temozolomide (90mg/kg/dose)
Fig. 4 illustrates the response of SC Implanted LOX IMVI Human Melanoma to Treatment with different doses of CTO with the Low Dose of Temozolomide (60mg/kg/dose)

### 5. DETAILED DESCRIPTION OF THE INVENTION

### CHEMOTHERAPY DOSING SCHEDULE INFLUENCES DRUG RESISTANCE

### DEVELOPMENT IN SEVERAL CANCERS

Drug resistance is the leading cause of chemotherapy failure in the treatment of cancer and is responsible for the death of a great majority of patients with metastatic late-stage cancer. Patients initially respond well to chemotherapy. However, cancer cells have significant plasticity and multiple cycles of chemotherapy have been shown to select tumor cells that are inherently resistant or that have developed resistance over the course of treatment and eventually, the disease becomes incurable.

### Chemotherapy Interference and Resistance in Tumor Microenvironment

Drug resistance can result from both tumor microenvironmental and molecular factors in cancer cells. Issues such as hypoxic regions, irregular blood flow and supply, the extracellular matrix, high density of cells within the tumor, high interstitial fluid pressure can create pharmacologic and physical barriers through which chemotherapeutic drugs cannot diffuse and reach the tumor. Alternatively drug delivery to tumor cells can be hindered by upregulation of cell membrane drug efflux transporters. The product of the MDR 1 gene, P-glycoprotein (P-gp) is a key molecule leading to cancer multidrug resistance. Mounting evidence suggests that bone-marrow-derived cells (BMDC) contribute to tumor growth, angiogenesis and metastasis. Importantly, in acute reactions to cytotoxic chemotherapy, several types of BMDCs are rapidly mobilized in tumors. These BMDC and plasma from chemotherapy treated mice promote metastatic properties in tumor cells by inducing matrix metalloproteinase -9 (MMP9) and epithelial-to -mesenchymal transition. In mice in which Lewis lung carcinoma cells were injected intravenously, treatment with paclitaxel, a cytotoxic agent, accelerated metastases in a manner that could be blocked by an MMP9 inhibitor. Therefore, inhibition of MMP9 by using a combinatorial regimen of paclitaxel and an MMP9 inhibitor could inhibit the metastases.

Similarly, some angiogenic therapies have been shown to paradoxically enhance vascular function. Targeting VEGF receptors using antibodies results in temporary improvement in vascular function, termed "vascular normalization" and is associated with a reduction in tumor hypoxia and increased radiation sensitivity. However, the transient nature of this response makes it difficult to translate into clinical practice. Over expression of VEGF is linked to oncogenic signaling and VEGF induction can be triggered by RAS signaling through phosphoinositide-3 kinase (PI3K) pathway. Signal transduction arising from PI3 kinase in particular plays a major role in cancer cell signaling. Therefore, combinatorial therapy with anti-VEGF, for example, CTO, may prevent the vascular improvement and improve the anti-angiogenic therapy. Similarly, PI3 kinase inhibitor, such as CTO, given with a chemotherapeutic cytotoxic may improve the delivery of the cytotoxic to the tumor and improve efficacy. CTO inhibits PI3 kinase activity by inhibiting conversion of PI2 to PI3. In addition, CTO is an inhibitor of calcium signaling and channeling which is important in the vertical targeting of PI3 kinase and in cancer cell proliferation. CTO is an inhibitor of VEGF which is required by malignant melanoma cells for growth. However, targeted therapy with VEGF blockers such as bevacizumab or Aflibercept® as monotherapy has failed to reduce the tumor burden and their combinations with cytotoxic drugs have failed in melanoma treatment.

### Chemotherapy Interference and Resistance due to Molecular Factors

Chemotherapy resistance occurs when cancers that have been responding to a therapy suddenly begin to grow. In other words, the cancer cells are resisting the effects of the chemotherapy. The drugs will need to be changed or the dose increased. There are several possible reasons for chemotherapy resistance at the molecular level. Some of the cancer cells that are not killed by the chemotherapy mutate and become resistant to the drug. Once they multiply they may be more resistant to the chemotherapy. Another reason is gene amplification. A cancer cell may produce hundreds of copies of a particular gene, which triggers an overproduction of proteins that render the chemotherapeutic drug ineffective. Yet another reason is that cancer cells may pump out of the cell the drug using p-glycoprotein or the cancer cell stops taking in drugs because the protein that transports the drug across the cell wall stops working. Another reason is that cancer cells develop mechanisms to inactivate the drug or to repair the DNA breaks caused by the drug. In other words, when treating cancer the best weapon is the smallest possible interference in the drug sensitivity and chemotherapy resistance.

Melanoma is a highly vascular tumor where VEGF is linked to pathogenesis and poor prognosis in melanoma patients. The molecular heterogeneity in melanoma makes it difficult to maintain the response of the BRAF inhibitors and it is suggested that PI3 kinase may limit the degree to which BRAF mutant melanoma remain sensitive to BRAF inhibitors.

Some drugs are toxic by themselves, but their toxicity may be potentiated when they are used in combination with other agents, the combination may be more toxic than the sum of the toxicities of the individual components. The idea that tissue damage associated with chemotherapy can activate a paracrine prosurvival secretory program suggests that inhibition of signaling pathways activated by IL-6 might potentiate the therapeutic efficacy of conventional chemotherapeutic cytotoxics.

### Examples of Chemotherapeutic Cytotoxic Drugs Demonstrating Drug Resistance

Temozolomide is an oral alkylating agent which has been used for the treatment of Grade IV astrocytoma, an aggressive brain tumor also known as glioblastoma multiforme. It is also used for treatment of melanoma a form of skin cancer, Grade III Anaplastic Astrocytoma and oligodendroglioma and malignant gliomas in adults and children. Laboratory studies and clinical trials are investigating whether it might be possible to further increase the anticancer potency of temozolomide by combining it with other agents.

### 6. EXAMPLES / COMPARATIVE EXAMPLES

### 1. Titration of Combinatorial Therapeutics in Normal Mice.

The purpose of the first experiment was to evaluate the tolerance of female athymic NCr-*nu*/*nu* mice to the combination treatment of 5-amino-1-(4-(4-chlorobenzoyl)-3,5-dichlorobenzyl)-1,2,3-triazole-4-carboxyamide orotate (CTO) and a cytotoxic chemotherapeutic agent selected from the group consisting of temozolomide, 5-FU, methotrexate, doxorubicin and daunorubicin.

Polyethylene glycol (PEG 400, MW 400) was purchased from Aldrich Chemistry. Deionized water was purchased from LabChem, Inc. Klucel (hydroxypropyl cellulose) was purchased from Aldrich. Saline (physiological saline solution, for animal use only) was purchased from Nova-Teck, Inc. Tween 80 (T80, polysorbate 80) was purchased from Fisher Scientific. CTO (MW 580.76) was stored at room temperature protected from light upon receipt. CTO was formulated once a week (on Days 1 and 8) at a concentration of 51.3 mg/mL in 40% PEG 400 in deionized water. Temozolomide® (temozolomide, Schering Co., 20 mg/capsule) was prepared on each day treatment by adding several drops of T80 to the powder, and then adding 0.3% Klucel in saline to yield a concentration of 4.5 mg/mL.

The study consisted of four groups of five mice per group for a total of 25 mice on Day 1. Animals in Group 1 were treated with CTO at a dose of 513 mg/kg/dose once a day for fourteen consecutive days (Q1Dx14, Days 1-14) in combination with temozolomide which was administered at a dose of 90 mg/kg/dose once every four days for a total of three treatments (Q4Dx3, Days 1, 5, and 9). Animals in Group 2 were treated with CTO at a dose of 342 mg/kg/dose on a Q1Dx14 schedule, in combination with temozolomide which was administered at a dose of 90 mg/kg/dose on a Q4Dx3 schedule. Animals in Group 3 were treated with CTO at a dose of 513 mg/kg/dose on a Q1Dx14 schedule, in combination with temozolomide which was administered at a dose of 60 mg/kg/dose on a Q4Dx3 schedule. Animals in Group 4 were treated with CTO at a dose of 342 mg/kg/dose on a Q1Dx14 schedule, in combination with temozolomide which was administered at a dose of 60 mg/kg/dose on a Q4Dx3 schedule. Individual body weights are presented in **Table 1.**

**Table 1 - Mean Body Weight (g)**

| Day | 1 | 4 | 8 | 11 | 15 | 18 | 22 |
|---|---|---|---|---|---|---|---|
| Group 1 | 21.9 | 20.6 | 20.8 | 21.2 | 22.6 | 23.6 | 24.3 |
| Group 2 | 22.4 | 22.0 | 21.4 | 21.9 | 23.4 | 24.1 | 25.1 |
| Group 3 | 23.7 | 22.3 | 22.8 | 23.0 | 25.2 | 26.3 | 26.9 |
| Group 4 | 24.4 | 23.0 | 23.7 | 24.4 | 25.3 | 25.9 | 26.5 |

**RESULTS-** Administration of CTO at a dose of 513 mg/kg/dose in combination with temozolomide at a dose of 90 or 60 mg/kg/dose (Groups 1 and 3, respectively) was associated with a maximum mean body weight loss of 6% (1.3-1.4 g), observed on Day 4. Administration of CTO at a dose of 342 mg/kg/dose in combination with temozolomide at a dose of 90 mg/kg/dose (Group 2) was associated with a maximum mean body weight loss of 4% (1.0 g), observed on Day 8. Administration of CTO at a dose of 342 mg/kg/dose in combination with temozolomide at a dose of 60 mg/kg/dose (Group 4) was associated with a maximum mean body weight loss of 6% (1.4 g), observed on Day 4. Animals in all four groups gained weight after the end of the treatment. Thus, the maximum tolerated dose (MTD) was above 513 mg/kg/dose for CTO and 90 mg/kg/dose for temozolomide in this experiment. MTD is defined as the dose which does not result in death or produces no more than 20% mean body weight loss.

### 2. Combinatorial Therapy with Temozolomide and CTO in Xenograft Model of human LOX IMVI Melanoma in mice

The purpose of the experiment TTI-6A was to evaluate the antitumor activity of CTO when administered in combination with temozolomide against subcutaneously (SC)-implanted human LOX IMVI melanoma xenografts in female, athymic NCr*-nu*/*nu* mice. Female six-week-old athymic NCr*-nu*/*nu* mice were purchased from Charles River Laboratories (Wilmington, MA). Human LOX IMVI melanoma cells were maintained as an ascites passage. Each mouse was implanted SC near the right flank with one million (1x10⁶) of the LOX IMVI human melanoma cell from an *in vivo* passage using a 23g needle. The day of tumor fragment implantation was designated as Day 0. Individual tumors of 90 animals grew to 126-198 mg in weight (126-198 mm³ in size) on Day 6 after tumor implantation (July 20, 2011), the day of treatment initiation. Those animals selected with tumors in the proper size range were assigned to nine treatment groups so that the mean tumor weights in all groups on Day 6 were as close to each other as possible (mean tumor weights being 165 or 166 mg, median tumor weights ranging from 162 to 176 mg).

CTO was administered to mice by exact individual animal's body weight on each day of treatment, with the injection volume being 0.1 mL/10 g body weight, temozolomide dosing formulations were administered to mice within 30 minutes of formulation by exact individual body weight on each day of treatment, with the injection volume being 0.2 mL/10 g body weight.

The study consisted of nine groups of ten mice per group. Animals in Group 1 were treated with the CTO vehicle (40% PEG 400 in deionized water) on a Q1Dx14 schedule (Days 6-19) in combination with the temozolomide vehicle (0.3% Klucel in saline), which was administered on a Q4Dx3 schedule (Days 6, 10, and 14). Animals in Groups 2 and 3 were treated with CTO at doses of 513 and 342 mg/kg/dose, respectively, on a Q1Dx14 schedule. Animals in Groups 4 and 5 were treated with temozolomide at doses of 90 and 60 mg/kg/dose, respectively, on a Q4Dx3 schedule. Animals in Groups 6 and 7 were treated with CTO on a Q1Dx14 schedule at doses of 513 and 342 mg/kg/dose, respectively, in combination with temozolomide which was administered at a dose of 90 mg/kg/dose on a Q4Dx3 schedule. Animals in Groups 8 and 9 were treated with CTO on a QlDxl4 schedule at doses of 513 and 342 mg/kg/dose, respectively, in combination with temozolomide which was administered at a dose of 60 mg/kg/dose on a Q4Dx3 schedule.

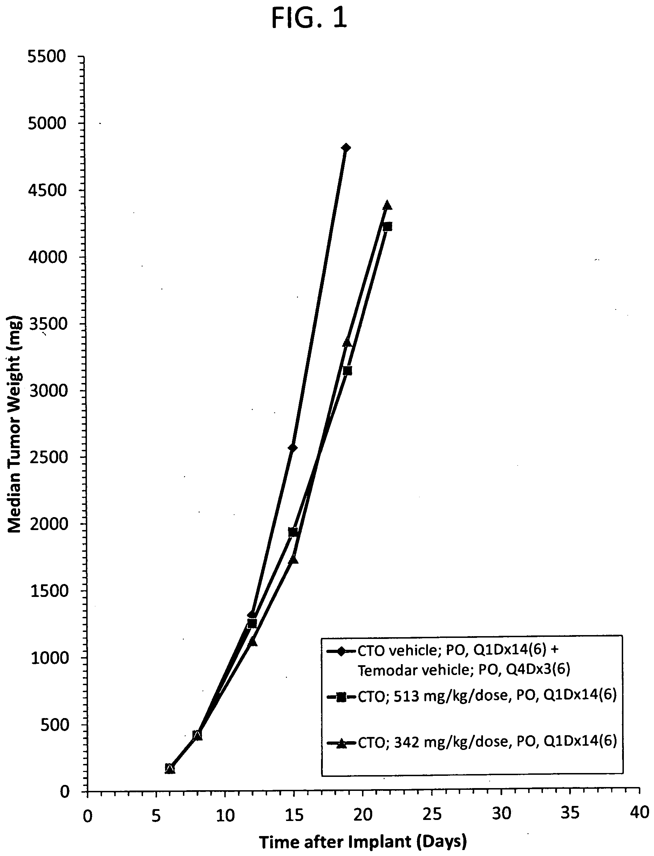

The SC tumors were measured and the animals were weighed two times a week. Tumor volume was determined by the formula for an ellipsoid sphere: L x W²/ 2 = mm³, where L and W refer to the larger and smaller perpendicular dimensions collected at each measurement. The experiment was terminated on Day 35.
**Parameters Evaluated-** Number of nonspecific deaths, number of complete tumor regressions, number of tumor-free survivors on Day 35, and the median time for the tumors to reach four tumor mass doublings were determined. Comparison of the median tumor weight in the treatment groups to the median tumor weight in the control group (T/C x 100%) on Day 19 (the last day of treatment with CTO and the last day of data collection when mice in the control group were still alive) was used for an additional evaluation of the antitumor efficacy of the combination treatments. Results are summarized in Figures 1-4. Oral administration of CTO at doses of 513 and 342 mg/kg/dose (Groups 2 and 3, respectively) was associated with maximum mean body weight losses of 4% (0.9 g) and 2% (0.6 g), respectively, observed on Day 8. Growth of the tumors in the groups treated with CTO at both doses was found to be statistically different from the growth of the tumors in the control group, when individual animal's times to reach four tumor mass doublings were compared (Group 1 vs. Group 2: *P*<0.001; Group 1 vs. Group 3: *P*=0.004). The T/C values on Day 19 were 65% and 70%, respectively. Tumor weights on Day 19 in both CTO-treated groups were statistically different from the tumor weights in the vehicles-treated control group (Group 1 vs. Group 2: *P*<0.001; Group 1 vs. Group 3: *P*=0.010). The observed antitumor activity was not dose-dependent when individual animal's times to reach four tumor mass doublings were compared (Group 2 vs. Group 3: *P*=0.556) and when individual animal's tumor weights on Day 19 were compared (Group 2 vs. Group 3: *P*=0.204). **Figure 1****.**

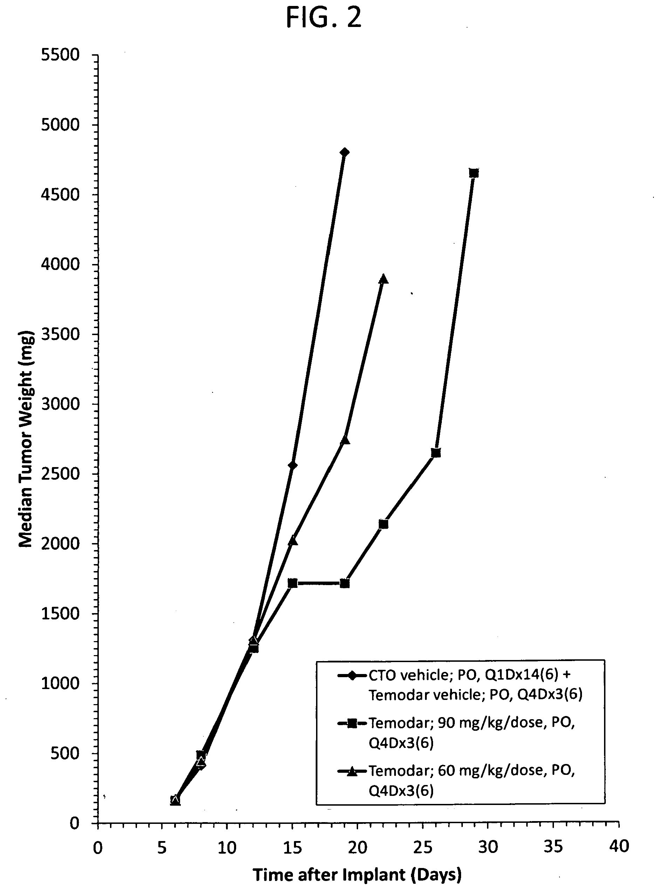

Oral administration of temozolomide at doses of 90 and 60 mg/kg/dose (Groups 4 and 5, respectively) was associated with maximum mean body weight loss of 7% (1.6-1.8 g) in both groups. The median tumor reached four tumor mass doublings in 17.2 and 12.3 days, producing a delay in the growth of the median tumor of 8.5 and 3.6 days, respectively. Growth of the tumors in both temozolomide-treated groups was found to be statistically different from the growth of the tumors in the control group, when individual animal's times to reach four tumor mass doublings were compared (*P*<0.001 for both groups). The T/C values on Day 19 were 36% and 57%, respectively. Tumor weights on Day 19 in both groups treated with temozolomide were statistically different from the tumor weights in the vehicles-treated control group (*P*<0.001 for both groups). The observed antitumor activity was dose-dependent when individual animal's times to reach four tumor mass doublings were compared (Group 4 vs. Group 5: *P*<0.001) and when individual animal's tumor weights on Day 19 were compared (Group 4 vs. Group 5: *P*=0.004). **Figure 2****.**

Administration of CTO at a dose of 513 mg/kg/dose in combination with temozolomide at a dose of 90 mg/kg/dose (Group 6) was associated with a maximum mean body weight loss of 16% (3.7 g), observed on Day 22. The median tumor reached four tumor mass doublings in 20.2 days, producing a delay in the growth of the median tumor of 11.5 days. Growth of the tumors in this combination treatment group was found to be statistically different from the growth of the tumors in the control group, when individual animal's times to reach four tumor mass doublings were compared (Group 1 vs. Group 6: *P*<0.001); however, tumor growth was not different from the growth of the tumors in the group treated with temozolomide at a dose of 90 mg/kg/dose alone (Group 4 vs. Group 6: P=0.383). The T/C value on Day 19 was 27%. Tumor weights on Day 19 in this combination group were statistically different from the tumor weights in the vehicles-treated control group (Group 1 vs. Group 6: P<0.001) and from the tumor weights in the group treated with temozolomide at a dose of 90 mg/kg/dose alone (Group 4 vs. Group 6: P=0.021).

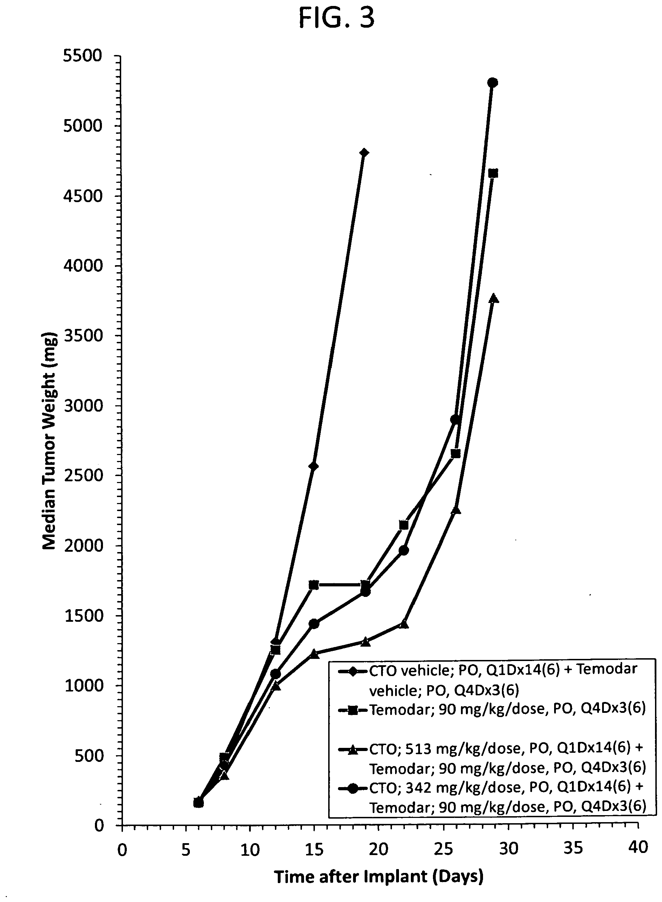

Administration of CTO at a dose of 342 mg/kg/dose in combination with temozolomide at a dose of 90 mg/kg/dose (Group 7) was associated with a maximum mean body weight loss of 15% (3.5 g), observed on Days 19 and 22. Three animals in the group died (with deaths occurring on Days 19, 25, and 28). The median tumor reached four tumor mass doublings in 16.9 days, producing a delay in the growth of the median tumor of 8.2 days. Growth of the tumors in this combination treatment group was found to be statistically different from the growth of the tumors in the control group, when individual animal's times to reach four tumor mass doublings were compared (Group 1 vs. Group 7: *P*<0.001); however, tumor growth was not different from the growth of the tumors in the group treated with temozolomide at a dose of 90 mg/kg/dose alone (Group 4 vs. Group 7: P=0.890). The T/C value on Day 19 was 35%. Tumor weights on Day 19 in this combination group were statistically different from the tumor weights in the vehicles-treated control group (Group 1 vs. Group 7: *P*<0.001); however, tumor weights were not different from the tumor weights in the group treated with temozolomide at a dose of 90 mg/kg/dose alone (Group 4 vs. Group 7: P=0.211). **Figure 3****.**

Administration of CTO at a dose of 513 mg/kg/dose in combination with temozolomide at a dose of 60 mg/kg/dose (Group 8) was associated with a maximum mean body weight loss of 8% (2.0 g), observed on Day 22. The median tumor reached four tumor mass doublings in 15.5 days, producing a delay in the growth of the median tumor of 6.8 days. Growth of the tumors in this combination treatment group was found to be statistically different from the growth of the tumors in the control group, when individual animal's times to reach four tumor mass doublings were compared (Group 1 vs. Group 8: *P*<0.001) and from the growth of the tumors in the group treated with temozolomide at a dose of 60 mg/kg/dose alone (Group 5 vs. Group 8: P=0.001). The T/C value on Day 19 was 47%. Tumor weights on Day 19 in this combination group were statistically different from the tumor weights in the vehicles-treated control group (Group 1 vs. Group 8: *P*<0.001) and from the tumor weights in the group treated with temozolomide at a dose of 60 mg/kg/dose alone (Group 5 vs. Group 8: P=0.017). The antitumor activity of the combination treatment was additive compared to the antitumor activity produced by the administration of each compound alone: a median tumor growth delay in Group 2 (CTO at a dose of 513 mg/kg/dose) = 3.1 days, a median tumor growth delay in Group 5 (temozolomide at a dose of 60 mg/kg/dose) = 3.6 day compared to a median tumor growth delay in Group 8 (CTO at a dose of 513 mg/kg/dose plus temozolomide at a dose of 60 mg/kg/dose) = 6.8 days.

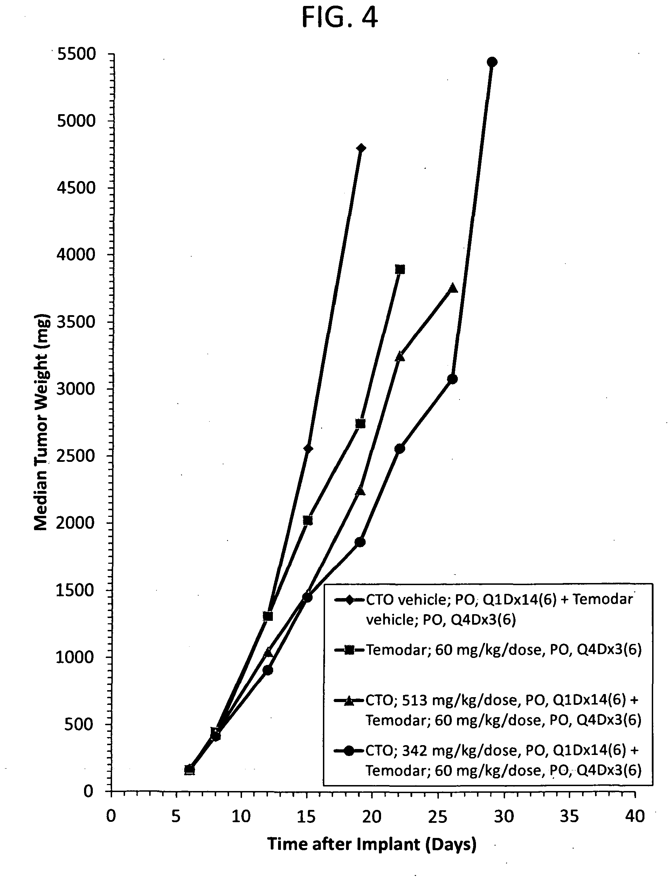

Administration of CTO at a dose of 342 mg/kg/dose in combination with temozolomide at a dose of 60 mg/kg/dose (Group 9, according to the invention) was associated with a maximum mean body weight loss of 10% (2.2-2.3 g), observed on Days 15 and 19. One animal died on Day 19. The median tumor reached four tumor mass doublings in 19.4 days, producing a delay in the growth of the median tumor of 10.7 days. Growth of the tumors in this combination treatment group was found to be statistically different from the growth of the tumors in the control group, when individual animal's times to reach four tumor mass doublings were compared (Group 1 vs. Group 9: *P*<0.001) and from the growth of the tumors in the group treated with temozolomide at a dose of 60 mg/kg/dose alone (Group 5 vs. Group 9: P<0.001). The T/C value on Day 19 was 39%. Tumor weights on Day 19 in this combination group were statistically different from the tumor weights in the vehicles-treated control group (Group 1 vs. Group 9: *P*<0.001) and from the tumor weights in the group treated with temozolomide at a dose of 60 mg/kg/dose alone (Group 5 vs. Group 9: P=0.001). The antitumor activity of the combination treatment was more than additive compared to the antitumor activity produced by administration of each compound alone: a median tumor growth delay in Group 3 (CTO at a dose of 342 mg/kg/dose) = 1.6 days, a median tumor growth delay in Group 5 (temozolomide at a dose of 60 mg/kg/dose) = 3.6 day compared to a median tumor growth delay in Group 9 (CTO at a dose of 342 mg/kg/dose plus temozolomide at a dose of 60 mg/kg/dose) = 10.7 days. **Figure 4****.**

Therefore, oral administration of CTO at doses of 513 or 342 mg/kg/dose for fourteen consecutive days resulted in a measurable inhibition of the growth of the human LOX IMVI melanoma xenografts when implanted SC in female athymic NCr*-nu*/*nu* mice. The observed antitumor activity of CTO was not dose-dependent. Oral administration of temozolomide at doses of 90 and 60 mg/kg/dose once every four days for a total of three treatments resulted in a measurable, dose-dependent inhibition of the growth of the human LOX IMVI melanoma xenografts. While the combination treatment of CTO at both doses tested with temozolomide at a dose of 90 mg/kg/dose did not result in an increased antitumor activity compared to the antitumor activity produced by the administration of temozolomide alone at a dose of 90 mg/kg/dose, the combination treatment of CTO at both doses tested with temozolomide at a dose of 60 mg/kg/dose resulted in an increased antitumor activity compared to the antitumor activity produced by the administration of temozolomide alone at a dose of 60 mg/kg/dose. While the antitumor activity of the combination treatment of CTO at a dose of 513 mg/kg/dose plus temozolomide at a dose of 60 mg/kg/dose was additive, the antitumor activity of the combination treatment of CTO at a dose of 342 mg/kg/dose plus temozolomide at a dose of 60 mg/kg/dose (according to the invention) was more than additive. These results demonstrate that the dose dilution strategy of using lower doses of temozolomide produced the maximum antitumor activity. This is contrary to the established practice of dose dense strategies currently used in treating cancer patients and promises to make a huge impact on achieving maximum treatment effects and lower toxicities through the "dose dilution" strategy.

## Claims

1. A composition comprising temozolomide for use in the treatment of melanoma wherein the treatment comprises administration orally of carboxyamidotriazole orotate and wherein the temozolomide is administered orally at a dose of 60 mg/kg/Q4Dx3, and wherein the carboxyamidotriazole orotate is administered at a dose of 342 mg/kg/day.

## Patentansprüche

1. Zusammensetzung, die Temozolomid für die Verwendung bei der Behandlung eines Melanoms umfasst, wobei die Behandlung die orale Verabreichung von Carboxyamidotriazolorotat umfasst und wobei das Temozolomid oral in einer Dosis von 60 mg/kg/Q4Dx3 verabreicht wird, und wobei das Carboxyamidotriazolorotat in einer Dosis von 342 mg/kg/Tag verabreicht wird.

## Revendications

1. Composition comprenant du témozolomide destiné à être utilisé dans le traitement de mélanomes, le traitement comprenant l'administration par voie orale d'orotate de carboxyamidotriazole, le témozolomide étant administré par voie orale à une dose de 60 mg/kg/Q4Dx3 et l'orotate de carboxyamidotriazole étant administré à une dose de 342 mg/kg/jour.
